(19) Europäisches Patentamt
European Patent Office
Office européen des brevets

(11) **EP 3 638 341 B1**

(12) **EUROPÄISCHE PATENTSCHRIFT**

(45) Veröffentlichungstag und Bekanntmachung des Hinweises auf die Patenterteilung:
**31.03.2021 Patentblatt 2021/13**

(21) Anmeldenummer: **18732722.6**

(22) Anmeldetag: **14.06.2018**

(51) Int Cl.:
**A61M 1/36** (2006.01)

(86) Internationale Anmeldenummer:
**PCT/EP2018/065891**

(87) Internationale Veröffentlichungsnummer:
**WO 2018/229229 (20.12.2018 Gazette 2018/51)**

(54) **VORRICHTUNG ZUR ZUGABE EINES ANTIKOAGULANS IN DAS BLUT EINES PATIENTEN**

DEVICE FOR ADDING AN ANTI-CLOTTING AGENT INTO A PATIENT'S BLOOD

DISPOSITIF SERVANT À AJOUTER UN ANTICOAGULANT AU SANG D'UN PATIENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.06.2017 DE 102017113061**

(43) Veröffentlichungstag der Anmeldung:
**22.04.2020 Patentblatt 2020/17**

(73) Patentinhaber: **Fresenius Medical Care Deutschland GmbH**
**61352 Bad Homburg (DE)**

(72) Erfinder:
• **WIESEN, Gerhard**
**61352 Bad Homburg (DE)**
• **SPICKERMANN, Reiner**
**97535 Burghausen (DE)**

(74) Vertreter: **Herrmann, Uwe**
**Lorenz Seidler Gossel**
**Rechtsanwälte Patentanwälte**
**Partnerschaft mbB**
**Widenmayerstraße 23**
**80538 München (DE)**

(56) Entgegenhaltungen:
**WO-A1-2016/168162     US-A- 6 017 318**
**US-A1- 2017 000 938**

**Beschreibung**

Die vorliegende Erfindung betrifft ein Verfahren und eine Vorrichtung zur Zugabe eines Antikoagulans in das Blut eines Patienten.

Ein Verabreichungssystem mit einer Rückkopplungs-Regelschleife ist beispielsweise aus der Druckschrift US6017318A bekannt. Zudem offenbart die Druckschrift US2017/000938A ein Blutbehandlungsgerät. Weiterhin offenbart die Druckschrift WO2016/168162 ein Infusionsgerät.

Während einer Dialysebehandlung wird das Blut des zu behandelnden Patienten mittels eines extrakorporalen Blutkreislaufs von dem Patienten abgezogen, durch einen Dialysator geführt und nach der darin erfolgten Blutbehandlung zurück zum Patienten geleitet. Die Durchleitung des Blutes durch den extrakorporalen Kreislauf wiederholt sich mehrfach, um das gewünschte Behandlungsziel zu erreichen.

Neben der Intimaverletzung durch die Dialysekanülen, mittels derer das Blut aus dem Gefäßsystem des Patienten abgezogen und diesem wieder zurückgeführt wird, kommt das Blut bei der Durchströmung des extrakorporalen Kreislaufs mit verschiedenen Materialien und großen Oberflächen - üblicherweise zwischen 1 m$^2$ und 2 m$^2$ - in Berührung. Darüber hinaus ist die Strömungsführung in dem extrakorporalen Blutkreislauf beispielsweise aufgrund von T-Stücken, der invasiven Blutdruckmessung über einen Transducerprotector etc. in dem Sinne nicht optimal, als dass Totzonen, Kanten, sonstige Strömungshindernisse sowie Stellen mit direktem Blut-Luft-Kontakt vorhanden sind.

All diese Störstellen können dazu führen, dass die Blutgerinnung aktiviert wird, was zur Folge hätte, dass die Behandlung abgebrochen werden muss, weil Blutgerinnsel beispielsweise den venösen Gerinnselfänger, der das Eindringen von Gerinnseln in den Blutkreislauf des Patienten verhindern sollen, blockiert, dass das Blut in den Kapillaren des Dialysators gerinnt etc.

Um eine Blutgerinnung und die damit verbundenen Nachteile zu vermeiden, ist es aus dem Stand der Technik bekannt, dem Blut ein Antikoaguians zuzugeben, bei dem es sich vorzugsweise um Heparin handelt.

Man unterscheidet zwei Typen von Heparin, das unfraktionierte Standardheparin mit Molekülgrößen zwischen 6.000 Da und 30.000 Da und das fraktionierte Low-Molecular-Weight Heparin (LMW-Heparin) mit Molekülgrößen zwischen 4.000 Da und 6.000 Da. In wässriger Lösung lassen sogenannte High-Flux-Dialysatormembranen fraktioniertes Heparin, d.h. LMW-Heparin mit einem Siebkoeffizienten von nahe 1 passieren, was einer fast 100 %-igen Elimination des Heparins über die Membran entspricht. Standardheparin passiert die Dialysatormembran mit einer geringfügig verringerten Wahrscheinlichkeit.

Im Gegensatz dazu ist Heparin (LMW-Heparin oder Standardheparin), das an Proteine gebunden ist, nicht dialysabel, d.h. es wird weder mittels Konvektion noch mittels Diffusion über die Dialysatormembran in die Dialysierflüssigkeit transportiert.

Zu Beginn der extrakorporalen Blutbehandlung besteht das Problem, dass ein erhöhter initialer Heparinverlust eintreten kann. Dieser ist darauf zurückzuführen, dass zu Beginn der Blutbehandlung der extrakorporale Blutkreislauf mit einer sogenannten Priming-Lösung gefüllt ist, d.h. mit einer physiologischen Kochsalzlösung oder mit Dialyselösung. Bei der beginnenden Durchmischung mit Blut ist die Proteinkonzentration und damit die Heparinbindungskapazität dementsprechend erniedrigt.

Hinzu kommt, dass die Dialysatormembran noch nicht mit einer Sekundärmembran aus Proteinen bedeckt ist (deren Ausbildung dauert ca. 20 - 30 min.).

Beides hat zur Folge, dass die Heparinzugabe in den extrakorporalen Blutkreislauf zu einem frühen Zeitpunkt dazu führt, dass ein großer Teil des zugegebenen Heparins über die Dialysatormembran übertritt und mit der Dialysierflüssigkeit, die die Dialysatormembran auf der anderen Membranseite überströmt, abtransportiert wird. Dieses Heparin steht dann nicht mehr für die Antikoaguiation des Blutes zur Verfügung.

Der vorliegenden Erfindung liegt die Aufgabe zugrunde, ein Verfahren und eine Vorrichtung bereitzustellen, mit denen sichergestellt ist, dass eine ausreichende Antikoagulans-Versorgung des Blutes vorliegt.

Diese Aufgabe wird durch ein Verfahren mit den Merkmalen des Anspruchs 1 und durch eine Vorrichtung mit den Merkmalen des Anspruchs 10 gelöst.

Danach ist vorgesehen, dass die Bolusapplikationszeit B von den Werten TBV und CO abhängt, wobei TBV das Blutvolumen des Patienten und CO das Herzminutenvolumen des Patienten darstellt.

Vorzugsweise ist vorgesehen, dass die Bolusapplikationszeit, d.h. die Zeitspanne, in der das Antikoagulans zugegeben wird, gleich oder größer ist als der Wert T = TBV/CO.

Durch die Bestimmung der Bolusapplikationszeit in Abhängigkeit der Größe T bzw. von TBV und CO wird erreicht, dass das Antikoagulans in einer Geschwindigkeit zugegeben wird, die an die innere Blutaustauschgeschwindigkeit angepasst ist.

Dies lässt es zu, dass das Antikoagulans weder zu schnell zugegeben wird, was den oben beschrieben Nachteil mit sich bringt, dass ein Teil des Antikoagulans über die Dialysatormembran entfernt wird, noch zu langsam zugegeben wird, was den Nachteil mit sich bringt, dass das Blut ggf. zur Antikoagulation neigt, da die Antikoagulations-Konzentration zu gering ist.

Das Antikoagulans wird mit der Rate $Q_{hep}$ = $V_{hep}$ / B appliziert, wobei $Q_{hep}$ das zu applizierende Volumenrate in Volumen/Zeiteinheit des Antikoagulans und $V_{hep}$ das zu applizierende Volumen des Antikoagulans darstellt.

Vorzugsweise handelt es sich bei dem Antikoagulans um Heparin, die Erfindung ist jedoch nicht auf dieses Antikoagulans beschränkt.

Denkbar ist es, dass die Zugabe des Antikoagulans mittels einer Pumpe erfolgt, vorzugsweise mittels einer Standard-Heparinpumpe.

Vorzugsweise ist des Weiteren vorgesehen, dass die Zugabe des Antikoagulans in den extrakorporalen Kreislauf eines Blutbehandlungsgerätes, insbesondere eines Dialysegerätes erfolgt.

Die Boluszugabe erfolgt vorzugsweise in Form eines Rechteckprofils mit konstanter Förderrate der Pumpe.

Der im Rahmen der Erfindung verwendete Begriff der Zugabe "in das Blut des Patienten" schließt somit sowohl den Fall ein, dass das Antikoagulans unmittelbar in den Blutkreislauf des Patienten injiziert wird, als auch den Fall, dass das Antikoagulans in den extrakorporalen Kreislauf eingeführt wird, der beispielsweise mit einer Priming-Lösung gefüllt ist.

In einer weiteren bevorzugten Ausgestaltung der Erfindung ist vorgesehen, dass die Zugabe des Antikoagulans vor der Blutbehandlung des Patienten, insbesondere vor der Dialysebehandlung des Patienten durchgeführt wird.

Der Wert TBV und/oder der Wert CO kann vor der Antikoagulans-Zugabe durch ein dem Fachmann bekanntes Verfahren ermittelt werden, was vergleichsweise aufwändig ist oder er kann aus einem Speicher ausgelesen werden, in dem diese Werte patientenindividuell abgelegt sind.

Durch die erfindungsgemäße Vorgehensweise wird erreicht, dass sich das Antikoagulans zügig und gleichmäßig über das gesamte Blutvolumen des Patienten verteilt. Durch die vorliegende Erfindung wird eine zu schnelle Zugabe von Antikoagulans verhindert, die zu dem oben beschriebenen Effekt führen würde, dass keine ausreichende Zeit für die Bindung des Antikoagulans an die Proteine des Blutes besteht, so dass das Antikoagulans im nicht gebundenen Zustand über die Membran des Dialysators übertritt und somit nicht mehr für den antikoagulativen Effekt zur Verfügung steht.

Der vorliegenden Erfindung liegt der Gedanke zugrunde, die Dauer der Zugabe des Antikoagulans so zu bemessen, dass neu mit einem Antikoagulans versehenes Blut in einem optimalen Verhältnis in den Blutkreislauf des Patienten dosiert wird.

Die Infusionsgeschwindigkeit des Antikoagulans wird an das Blutvolumen und an den "Cardiac Output" (CO), d.h. an das Herzminutenvolumen des Patienten und damit an dessen individuelle innere Blutaustauschgeschwindigkeit angepasst. Das Herzminutenvolumen oder auch Herzzeitvolumen ist das Volumen des Blutes, das pro Zeiteinheit vom Herzen des Patienten gefördert wird. Es wird üblicherweise in der Einheit l/min angegeben.

Das Herzminutenvolumen ist u.a. abhängig von der Herzfrequenz und liegt bei erwachsenen Personen im Ruhezustand üblicherweise im Bereich von 4,5 - 5 l/min.

Sowohl das Herzminutenvolumen als auch das Blutvolumen des Patienten können mittels dem Fachmann bekannter Methoden ermittelt werden.

Beispiele sind die Echokardiographie oder die indirekte Berechnung, wie z.B. mittels des Arterial-Stiffness-Monitor, Verdünnungsmethoden (Boli), Blutvolumenmonitor (BVM), Bluttemperaturmonitor (BTM), Thermodilution etc.

Da die Bestimmung des CO zeitaufwändig ist, ist es auch denkbar, den patientenspezifischen CO-Wert als "Ruhe-CO-Wert" in einem Speicher zu hinterlegen. Dies kann beispielsweise im Speicher des Dialysegerätes oder eines sonstigen Behandlungsgerätes, in einer Patientendatei, auf einer Patientenkarte etc. erfolgen.

Die vorliegende Erfindung betrifft des Weiteren eine Vorrichtung zur Zugabe eines Antikoagulans in das Blut eines Patienten, wobei die Zugabe in Form eines Bolus erfolgt, wobei die Vorrichtung Mittel zur Bestimmung der Bolusapplikationszeit B in Abhängigkeit der Werte TBV und CO aufweist, wobei TBV das Blutvolumen des Patienten und CO das Herzminutenvolumen des Patienten darstellt und wobei die Vorrichtung des Weiteren Mittel zur Verabreichung des Antikoagulans innerhalb der bestimmten Bolusapplikationszeit aufweist.

Vorzugsweise ist vorgesehen, dass die Mittel zur Bestimmung der Bolusapplikationszeit derart ausgeführt sind, dass die Beziehung $B \geq T$ erfüllt ist, dass also die Bolusapplikationszeit dem Wert T entspricht oder diesen übersteigt.

Die Vorrichtung kann Mittel zur Bestimmung von TBV und/oder von CO aufweisen.

Alternativ oder zusätzlich kann ein Speicher vorgesehen sein, auf den die Vorrichtung Zugriff hat bzw. der einen Bestandteil der Vorrichtung bildet und in dem die Werte für TBV und/oder CO abgespeichert sind, so dass aus den gespeicherten Werten T patientenindividuell berechnet werden kann.

Die Mittel zur Verabreichung des Antikoagulans sind beispielsweise als Pumpe ausgeführt, wobei die Pumpe vorzugsweise derart angeordnet ist, dass diese das Antikoagulans in den extrakorporalen Blutkreislauf eines Blutbehandlungsgerätes fördert.

Wie oben ausgeführt, handelt es sich bei dem Antikoagulans vorzugweise um Heparin, jedoch können auch andere Antikoagulantien zum Einsatz kommen.

Die erfindungsgemäße Vorrichtung kann Bestandteil eines Blutbehandlungsgerätes, vorzugsweise eines Dialysegeräts sein oder in geeigneter Weise mit einem Blutbehandlungsgerät, vorzugsweise mit einem Dialysegerät in Verbindung stehen.

Vorzugsweise ist vorgesehen, dass der Fachmann bei der Verabreichung des Bolus des Antikoagulans bzw. bei der Bolusapplikationszeit auch die Blutverteilung im Körper des Patienten, die Tot-, Vorlauf- und Nachlaufzeiten der Antikoagulans-Pumpe, die Clearance des Antikoagulans, die technischen Daten des Filters bzw. des Dialysators und die diffusiven und konvektiven Transportmechanismen über den Filter/Dialysator berücksichtigt.

Bei dem verabreichten Heparin kann es sich beispielsweise um unfraktioniertes hochmolekulares Heparin oder um LMW-Heparin handeln.

Die vorliegende Erfindung betrifft des Weiteren ein Blutbehandlungsgerät, insbesondere ein Blutbehandlungsgerät mit einem extrakorporalen Blutkreislauf und besonders bevorzugt ein Dialysegerät.

Weitere Einzelheiten und Vorteile der Erfindung werden anhand eines in der Zeichnung dargestellten Ausführungsbeispiels näher erläutert.

Es zeigen:

Figur 1:      eine schematische Ansicht eines an einem Dialysegerät angeschlossenen Patienten und

Figur 2:      eine schematische Darstellung der Flüsse der Anordnung gemäß Figur 1.

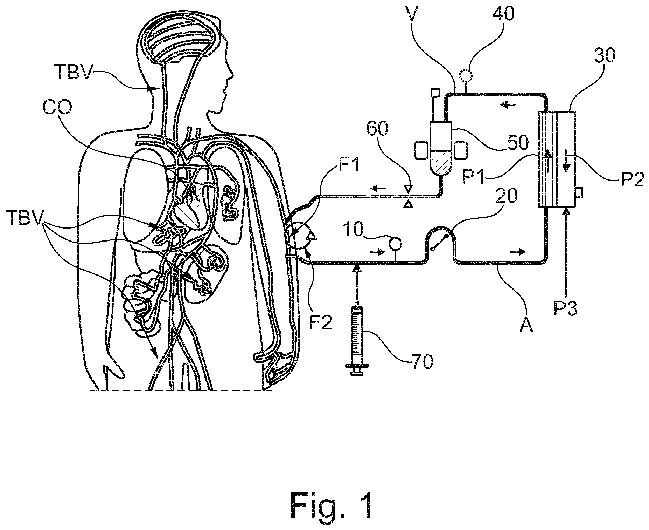

Wie aus Figur 1 ersichtlich, ist der Patient mittels einer Fistel an einen extrakorporalen Blutkreislauf eines Dialysegerätes angeschlossen, dessen arterieller Zweig mit dem Bezugszeichen A und dessen venöser Zweig mit dem Bezugszeichen V gekennzeichnet ist. Die Flussrichtung des Blutes durch den extrakorporalen Blutkreislauf ist durch Pfeile angegeben.

Das Blut gelangt durch den arteriellen Zweig A, in dem sich die Druckmesseinrichtung 10 und die Blutpumpe 20 befinden, zu dem Dialysator 30 und von diesem in den venösen Zweig V. In diesem befindet sich die venöse Druckmesseinrichtung 40 und die venöse Tropfkammer 50. Stromabwärts von der Tropfkammer 50 ist die venöse Klemme 60 angeordnet. Nach dem Passieren dieser Klemme gelangt das Blut zurück zum Patienten.

In dem Dialysator 30 ist der Blutfluss durch den Pfeil P1 gekennzeichnet und der Dialysatfluss durch den Pfeil P2. Blut und Dialysat sind durch eine oder mehrere semipermeable Membranen, vorzugsweise durch ein Hohlfaserbündel voneinander getrennt. Wie dies aus den Pfeilen P1 und P2 hervorgeht, wird der Dialysator 30 in dem dargestellten Ausführungsbeispiel im Gegenstrom von Blut und Dialysat durchströmt.

Der Pfeil P3 kennzeichnet die Gefahr des Heparinverlustes über die Membran des Dialysators, die insbesondere dann besteht, wenn das Heparin noch nicht an die Proteine des Blutes gebunden ist, etwa weil es mit zu großer Geschwindigkeit bzw. binnen zu kurzer Zeit zugegeben wurde.

Das Bezugszeichen F1 kennzeichnet den Fistelfluss und der Kreis im Bereich der Fistel eine mögliche Fistel-Rezirkulation F2.

Mit dem Bezugszeichen 70 ist eine Heparin-Pumpe gekennzeichnet, die wie aus Figur 1 ersichtlich mit dem arteriellen Zweig A in Verbindung steht und das Heparin in diesen Zweig fördert.

Die dargestellten Elemente des Blutkreislaufes werden durch eine nicht dargestellte Steuer- oder Regelungseinheit des Dialysegerätes angesteuert.

Das Bezugszeichen CO kennzeichnet das Herzminutenvolumen und das Bezugszeichen TBV das Gesamtblutvolumen des Patienten.

In dem hier dargestellten Beispiel ist der Blutfluss $Q_B$ während der Behandlung 300 ml/min und der Fistelfluss 1 ml/min.

Weiterhin ist in dem dargestellten Beispiel CO 5 l/min und TBV 5 l.

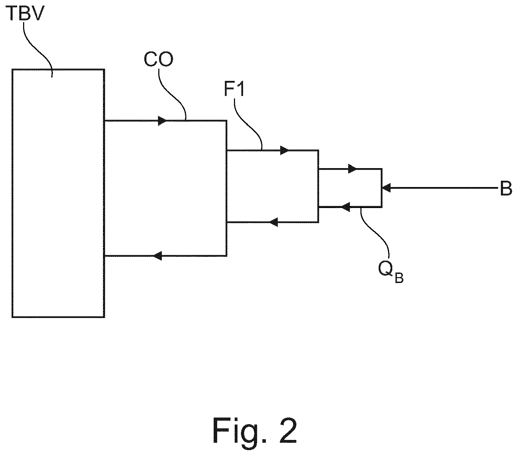

Diese exemplarischen Werte sowie die schematischen Flüsse sind in Figur 2 nochmals dargestellt.

Nach der oben genannten Formel ergibt sich für T = TBV/CO = 1 min.

Erfindungsgemäß ist die Bolusapplikationsdauer B für das Antikoagulans somit auf einen Wert von 1 min oder mehr, vorzugsweise auf 2 min einzustellen, d.h. das Antikoagulans wird mittels der Pumpe 70 über eine Dauer von z.B. 2 min in den extrakorporalen Kreislauf zugegeben. Diese Zugabe erfolgt vorzugsweise vor Beginn der Dialysebehandlung.

Die Berechnung von B erfolgt in einer Recheneinheit des Dialysegerätes, die auf einen Speicher zugreift, in dem die Werte für TBV und CO patientenindividuell gespeichert sind.

Auch ist es denkbar, dass das Dialysegerät eine Leseeinrichtung aufweist, die diese Werte von einer Patientenkarte oder dergleichen einliest.

Nach der Zugabe des Antikoagulans beginnt die Blutbehandlung durch Einschalten der Blutpumpe 20 sowie der nicht dargestellten Dialysatpumpe.

Geht man davon aus, dass $V_{hep}$ das zuzugebende Volumen von Heparin ist, ergibt sich für die Flussrate $Q_{hep}$, mit der die Pumpe 70 fördert:

$$Q_{hep} = V_{hep} / B = V_{hep} / 2\ min.$$

Diese Soll-Fördergeschwindigkeit wird der Pumpe 70 von der Steuer- oder Regelungseinheit bzw. von der genannten Recheneinheit des Dialysegerätes angegeben.

**Patentansprüche**

1.    Vorrichtung zur Zugabe eines Antikoagulans in das Blut eines Patienten, wobei die Zugabe in Form eines Bolus erfolgt, **dadurch gekennzeichnet, dass** die Vorrichtung Mittel zur Bestimmung der Bolusapplikationszeit B in Abhängigkeit der Werte TBV und CO aufweist, wobei TBV das Blutvolumen des Patienten und CO das Herzminutenvolumen des Patienten darstellt, und wobei die Vorrichtung des Weiteren Mittel zur Verabreichung des Antikoagulans innerhalb der bestimmten Bolusapplikationszeit B aufweist.

2.    Vorrichtung nach Anspruch 1, **dadurch gekennzeichnet, dass** die Mittel zur Bestimmung der Bolusapplikationszeit derart ausgeführt sind, dass die Beziehung B ≥ T erfüllt ist.

3. Vorrichtung nach Anspruch 1 oder 2, **dadurch gekennzeichnet, dass** die Vorrichtung Mittel zur Bestimmung von TBV und/oder von CO aufweist und/oder dass ein Speicher vorgesehen ist, in dem die Werte für TBV und/oder CO abgespeichert sind und auf den durch die Vorrichtung zugreifbar ist oder der Bestandteil der Vorrichtung ist.

4. Vorrichtung nach einem der Ansprüche 1 bis 3, **dadurch gekennzeichnet, dass** die Mittel zur Verabreichung des Antikoagulans als Pumpe (70) ausgeführt sind, wobei die Pumpe (70) vorzugsweise derart angeordnet ist, dass diese das Antikoagulans in den extrakorporalen Blutkreislauf eines Blutbehandlungsgerätes fördert und/oder dass es sich bei dem Antikoagulans um Heparin handelt.

5. Vorrichtung nach einem der Ansprüche 1 bis 4, **dadurch gekennzeichnet, dass** die Vorrichtung Bestandteil eines Blutbehandlungsgerätes, vorzugsweise eines Dialysegeräts, ist oder mit einem Blutbehandlungsgerät, vorzugsweise mit einem Dialysegerät in Verbindung steht.

6. Blutbehandlungsgerät, insbesondere Dialysegerät mit wenigstens einer Vorrichtung gemäß einem der Ansprüche 1 bis 5.

## Claims

1. Apparatus for adding an anticoagulant to the blood of a patient, wherein the addition takes place in the form of a bolus, **characterized in that** the apparatus comprises means for determining the bolus application time B in dependence on the values TBV and CO, wherein TBV represents the blood volume of the patient and CO the cardiac output of the patient, and wherein the apparatus furthermore comprises means for administering the anticoagulant within the specific bolus application time B.

2. Apparatus in accordance with claim 1, **characterized in that** the means for determining the bolus application time are configured such that the relationship B ≥ T is satisfied.

3. Apparatus in accordance with claim 1 or 2, **characterized in that** the apparatus comprises means for determining TBV and/or CO and/or **in that** a memory is provided in which the values for TBV and/or CO are stored and which the apparatus can access or which is a component of the apparatus.

4. Apparatus in accordance with one of the claims 1 to 3, **characterized in that** the means for administering the anticoagulant are configured as a pump (70), wherein the pump (70) is preferably arranged such that it conveys the anticoagulant into the extracorporeal blood circuit of a blood treatment machine and/or **in that** the anticoagulant is heparin.

5. Apparatus in accordance with one of the claims 1 to 4, **characterized in that** the apparatus is a component of a blood treatment machine, preferably of a dialysis machine, or is connected to a blood treatment machine, preferably to a dialysis machine.

6. Blood treatment device, in particular a dialysis machine, having at least one apparatus in accordance with one of the claims 1 to 5.

## Revendications

1. Dispositif servant à ajouter un anticoagulant au sang d'un patient, dans lequel l'ajout se fait sous la forme d'un bolus, **caractérisé en ce que** le dispositif présente des moyens pour définir le temps d'application de bolus B en fonction des valeurs TBV ou CO, dans lequel TBV représente le volume sanguin du patient et CO représente le débit cardiaque du patient, et dans lequel le dispositif présente par ailleurs des moyens pour l'administration de l'anticoagulant dans le temps d'application de bolus B défini.

2. Dispositif selon la revendication 1, **caractérisé en ce que** les moyens pour définir le temps d'application de bolus sont réalisés de telle manière que le rapport B ≥ T est rempli.

3. Dispositif selon la revendication 1 ou 2, **caractérisé en ce que** le dispositif présente des moyens pour définir le TBV et/ou le CO, et/ou qu'une mémoire est prévue, dans laquelle les valeurs du TBV et/ou du CO sont sauvegardées et qui peut être consultée par le dispositif ou qui fait partie intégrante du dispositif.

4. Dispositif selon l'une quelconque des revendications 1 à 3, **caractérisé en ce que** les moyens pour l'administration de l'anticoagulant sont réalisés en tant que pompe (70), dans lequel la pompe (70) est disposée de préférence de telle manière que celle-ci refoule l'anticoagulant dans le circuit sanguin extracorporel d'un appareil de traitement du sans et/ou que l'anticoagulant est de l'héparine.

5. Dispositif selon l'une quelconque des revendications 1 à 4, **caractérisé en ce que** le dispositif fait partie intégrante d'un appareil de traitement du sang, de préférence d'un appareil de dialyse, ou est relié à un appareil de traitement du sang, de préférence à un appareil de dialyse.

6.  Appareil de traitement du sang, en particulier appareil de dialyse, avec au moins un dispositif selon l'une quelconque des revendications 1 à 5.

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6017318 A
- US 2017000938 A
- WO 2016168162 A